# EUROPEAN PATENT APPLICATION

(11) **EP 4 789 694 A1**
(43) Date of publication of application: **12.08.2026**
(21) Application number: 25305772.3
(22) Date of filing: 28.05.2025
(51) Int. Cl.: A61L 31/02, A61L 31/10, A61L 31/16

(54) **FIBRIN COATINGS FOR THROMBECTOMY DEVICES**

(30) Priority: 07.02.2025 EP 25305184
(71) Applicant: Balt Innovation, 95160 Montmorency (FR)
(72) Inventor: GUYOT, Diane, 95160 Montmorency (FR); KERSANI, Dyhia, 95160 Montmorency (FR)
(74) Representative: Icosa

(57) **Abstract**

Medical devices, including insertable/implantable medical devices, may include proteins bound to a nickel titanium surface of the medical device. In some cases, the medical device may include a protein (e.g., a fibrin-binding protein) coating a surface of the medical implant. For example, the protein may be covalently bound via a covalent ester bond directly to the nickel titanium surface of the medical device. Also described herein are methods of making and using such medical devices.

## Description

### BACKGROUND

Ischemic stroke occurs when a vessel supplying blood to the brain is obstructed. In 87% of all strokes, thrombus or blood clot develops in a blood vessel. Mechanical thrombectomy is a primary treatment for ischemic stroke. Most strokes are ischemic, caused by a blocked blood vessel in the brain that cuts oxygen supply resulting in a neurological deficit. Ischemic stroke can often be treated with the clot-dissolving drug, e.g., a thrombolytic (TPA), also referred to as a "clot buster," or other advanced interventions when treatment is sought at the earliest signs of stroke. Less common is hemorrhagic stroke in which a blood vessel ruptures causing bleeding into or around the brain. According to the National Stroke Association, 2 million brain cells die for every minute of stroke, increasing a patient's chances every 60 seconds for suffering permanent, life-changing disabilities.

Treatment administered within the "Golden Hour," or first 60 minutes of stroke symptoms onset regularly leads to better patient outcomes, including fewer or even no lasting deficits. However, for the majority of ischemic stroke patients, the treatment window for the brain-saving tPA drug is only three hours. Patients who receive tPA within 60-90 minutes of stroke symptoms onset have the best possible chance for full recovery. According to medical data, the benefits of tPA are halved for every 90-minute delay in seeking medical treatment.

Endovascular mechanical thrombectomy is a primary treatment for acute ischemic stroke. Stentrievers are self-expanding stents that are endovascularly delivered through microcatheters. They are delivered to the site of an occlusion with the intended purpose of engaging and removing the clot thus restoring intracranial flow, known as revascularization.

The human cost of lasting effects of stroke, including memory loss, movement loss, vision problems, dysphagia, fatigue, and speech problems inform the need for better solutions to aid stroke patients. Described herein are devices and methods that may address these or other stroke effects.

### SUMMARY OF THE DISCLOSURE

Described herein are methods and apparatuses, including compositions, to improve the effectiveness of medical devices including, but not limited to, clot retrieval devices, coils (e.g., occlusive coils), stents, etc., by coating (functionalizing) the medical device. In particular, described herein are methods and compositions for directly attaching proteins, including antibodies, to a nickel titanium (e.g., NITINOL) medical device without the need for any intermediate molecule. Although the examples described herein specifically describe coating medical devices with proteins that target blood clots, e.g., fibrin, these methods and compositions may be applied to virtually any protein.

The methods (and the resulting apparatuses) described herein may include treating the metallic surface of a medical device to generate chemical groups that will allow the direct conjugation of a protein, such as an anti-fibrin antibody or peptide to the surface, resulting in organometallic devices with bioactive surfaces. These methods and apparatuses are different than conventional coatings or embedding strategies, as described in greater detail herein.

In some examples a method of forming a medical device as described herein may include first activating the nickel titanium surface of the medical device, e.g., with sodium hydroxide, to hydroxylate the nickel titanium to deprotonate the native hydroxyls on the surface of the titanium oxide, generating Ti-OH groups, and then activating carboxyl groups (-COOH) on the protein (e.g., antibody) to form an activated amide complex, which is then reacted with the OH group on the medical device surface to form a covalent ester bond. The carboxyl groups may be activated by a carbodiimide such as 1-Ethyl-3-(3-dimethylaminopropyl)carbodiimide (EDC) and may be stabilized by N-hydroxy succinimide (NHS).

For example, described herein medical devices having a fibrin-binding protein covalently linked to the nickel titanium outer surface of the medical device. Specifically, described herein are nickel titanium stents with a fibrin-binding protein covalently linked to the nickel titanium outer surface of the stent, as well as methods of making (coating) these stents. The fibrin-binding protein may be an antibody (e.g., an anti-fibrin antibody, such as an IgG1 antibody including anti-human fibrin antibody 59D8), or a Fab portion of an antibody), or other protein having a specific binding affinity for fibrin, such as a CREKA polypeptide (a synthetic pentapeptide of Cys-Arg-Glu-Lys-Ala). The medical device surface may be coated with the fibrin-binding protein within a desired range, such as, for example between about 20 nM to 1 µM (e.g., between about 50 nM to 900 nM, between about 100 nM to 800 nM, between about 20 nM to 600 nM, between about 100 nM to 1 µM, 100 nM or greater, 150 nM or greater, 200 nM or greater, 250 nM or greater, 300 nM, etc.). For example, the surface may be coated with a fibrin-binding protein at a concentration of between about 0.1 µg/ml and 500 µg /ml (e.g., between about 0.1 µg /ml and 200 µg/ml, between about 0.1 µg /ml and 150 µg/ml, between about 0.1 µg/ml and 100 µg/ml, between about 1 µg/ml and 300 µg /ml, between about 1 µg/ml and 250 µg/ml, between about 5 µg/ml and 200 µg/ml, between about 5 µg/ml and 150 µg/ml, between about 10 µg /ml and 200 µg/ml, between about 20 µg/ml and 200 µg/ml, between about 100 µg/ml and 500 µg/ml, between about 200 µg/ml and 500 µg/ml, etc.).

For example, described herein are endovascular clot-retrieval devices comprising: a body region comprising a surface formed of a nickel titanium alloy; and a fibrin binding protein covalently bound via a covalent ester bond directly to the surface formed of the nickel titanium alloy. In any of these methods and apparatuses, the fibrin binding protein may selectively bind fibrin over fibrinogen. The fibrin binding protein may comprise an antibody that specifically binds fibrin. The fibrin binding protein may comprise a 59D8 antibody. In any of these methods and apparatuses, the fibrin binding protein may comprise a peptide that specifically binds fibrin. In any of these methods and apparatuses, the fibrin binding protein comprises a CREKA peptide. The clot-retrieval device may comprise a stent (e.g., a single stent or a stentriever). In any of these methods and apparatuses the clot-retrieval device may comprise a self-expanding nickel-titanium body.

Also described herein are endovascular clot-retrieval devices comprising: a body region; and a coating of a fibrin binding agent on the body region. In any of these methods and apparatuses, the fibrin binding agent selectively binds fibrin over fibrinogen. The fibrin binding agent may comprise an antibody that specifically binds fibrin. In any of these methods and apparatuses, the fibrin binding agent may comprise a 59D8 antibody. The fibrin binding agent may comprise a peptide that specifically binds fibrin, preferably a CREKA peptide. In any of these methods and apparatuses, the clot-retrieval device may comprise a stent (e.g., a stentriever). The clot-retrieval device may comprise a self-expanding NITINOL body. The coating may comprise a polydopamine (PDA) coating.

Also described herein are methods of forming an implantable medical device, for example, the method may include: functionalizing a nickel titanium surface of a body of an implantable medical device with a protein by: exposing the surface to a sodium hydroxide (NaOH) solution to hydroxylate the surface, rinsing the surface, and incubating the surface with a protein in a 2-Morpholinoethanesulfonic acid (MES) buffer to covalently link the protein to the nickel titanium surface. In any of these methods, exposing the surface to the NaOH solution to hydroxylate the surface comprises exposing the surface to a solution of between 1M and 9M NaOH at between 70 and 90 degrees C for between 20 minutes and 3 hours. In any of these methods and apparatuses, exposing the surface to the NaOH solution comprises exposing the surface to a 5M NaOH solution at 80 degrees C for 1 hour. The protein may be any appropriate protein, including, but not limited to a fibrin binding agent. The protein may be an antibody. Any of these methods may include a 59D8 antibody. In some cases the protein comprises a CREKA peptide. Any of these methods may optionally include incubating the protein with NHS (N-hydroxysuccinimide) and EDC (1-ethyl-3-(3-dimethylaminopropyl) carbodiimide). The implantable medical device may include a stent (e.g., a stentriever).

Also described herein are methods of removing a clot from a body, the method may comprise: inserting a clot retrieval device into a blood vessel lumen, wherein the clot retrieval device comprises a fibrin binding protein bound via a covalent ester bond directly to a nickel titanium surface of the clot retrieval device; attaching, by the fibrin binding protein, the clot to the clot retrieval device; and removing the clot from the body. After inserting the clot retrieval device, the method may include expanding the clot retrieval device in the blood vessel lumen. In any of these methods, prior to inserting the clot retrieval device, the method may include identifying that the patient has a clot blockage. The fibrin binding agent may comprise a fibrin binding peptide, and attaching comprises attaching the clot to the fibrin binding peptide. The fibrin binding agent may comprise an anti-fibrin antibody, and attaching comprises attaching the clot to the anti-fibrin antibody.

Also described herein are methods of removing a clot from blood, comprising: inserting a clot retrieval device comprising a coating of a fibrin binding agent into a blood vessel lumen; and attaching, by the fibrin binding agent, the clot to the clot retrieval device. The method may further include, after the inserting a clot retrieval device step, expanding the clot retrieval device in the blood vessel lumen. The method may include, prior to the inserting step, identifying that the patient is suspected of having a clot blockage. The fibrin binding agent may comprise a fibrin binding peptide, and the step of attaching comprises attaching the clot to the fibrin binding peptide. The fibrin binding agent may comprise an anti-fibrin antibody, and the step of attaching may comprise attaching the clot to the anti-fibrin antibody.

Also described herein are methods of forming a medical device, the method comprising: deprotonating native and/or induced hydroxyl's on a nickel titanium surface of the medical device to form reactive hydroxyl group; activate a carboxyl group (-COOH) on a protein to form an activated amide complex and stabilizing the activated amide complex; forming a covalent ester bond between the reactive hydroxyl group and the activated amide complex to form a covalent ester bond linking the protein to the nickel titanium surface. Deprotonating native and/or induced hydroxyl's on the nickel titanium surface may comprise exposing the nickel titanium surface to a NaOH solution. Activating the carboxyl group (-COOH) may comprise using 1-ethyl-3-(3-dimethylaminopropyl) carbodiimide (EDC) to activate the carboxyl group. Any of these methods may include using N-hydroxy succinimide (NHS) to stabilize the activated amide complex. As mentioned, in any of these methods and apparatuses, the protein may comprise an antibody that specifically binds fibrin. For example, the protein may comprise a 59D8 antibody. In some cases the protein comprises a CREKA peptide. The medical device may be any appropriate medical device including a nickel titanium material. For example, the medical device may comprise a coil. The medical device may comprise a stent. The medical device may comprise a clot retriever.

A method of forming an implantable medical device may include: coating a body of an implantable medical device with a coating of a fibrin binding agent, wherein the coating is between 0.1 µm and 500 µm thick. The coating may target fibrin but may not appreciably bind to fibrinogen. The method may include incubating the coated body at an alkaline pH. Incubating the coated body at an alkaline pH may comprise incubating in a bicarbonate buffer having a pH of about 9. Coating the body may comprise coating with a monoclonal antibody against fibrin, preferably a 59D8 antibody. Coating the body may comprise coating with a peptide that specifically binds fibrin, preferably a CREKA peptide. In some examples coating comprises dip coating the body. In some cases coating comprises coating in a polydopamine (PDA) coating. Coating the body of the implantable medical device may comprise coating onto a nickel-titanium surface. Coating may comprise coating in a polydopamine (PDA) coating and then coating with an anti-fibrin antibody or antibody binding fragment. Coating may comprise coating with NHS (N-hydroxysuccinimide) and EDC (1-ethyl-3-(3-dimethylaminopropyl) carbodiimide) and anti-fibrin antibody, anti-fibrin antibody binding fragment or a fibrin binding agent. For example, coating may comprise covalently attaching the fibrin binding agent to the implantable medical device.

Also described herein are methods of treating an aneurysm, comprising: inserting an insertion device into a blood vessel in need of treatment, wherein the insertion device carries a clot retrieval device comprising a coating of a fibrin binding agent; and removing the insertion device to thereby leave the clot retrieval device in the blood vessel. Any of these methods may include attaching, by the fibrin binding agent, clot to the clot retrieval device. Coating may comprise a fibrin binding protein covalently bound via a covalent ester bond directly to a nickel titanium surface of the clot retrieval device.

All of the methods and apparatuses described herein, in any combination, are herein contemplated and can be used to achieve the benefits as described herein.

The invention relates to an endovascular clot-retrieval device, the device comprising:
a body region comprising a surface formed of a nickel titanium alloy; and
a fibrin binding protein covalently bound via a covalent ester bond directly to the surface formed of the nickel titanium alloy.

In some embodiments, the fibrin binding protein selectively binds fibrin over fibrinogen.

In some embodiments, the fibrin binding protein comprises an antibody that specifically binds fibrin.

In some embodiments, the fibrin binding protein comprises a 59D8 antibody.

In some embodiments, the fibrin binding protein comprises a peptide that specifically binds fibrin.

In some embodiments, the fibrin binding protein comprises a CREKA peptide.

In some embodiments, the clot-retrieval device comprises a stentriever.

In some embodiments, the clot-retrieval device comprises a self-expanding nickel-titanium body.

The invention relates also to an endovascular clot-retrieval device comprising:
a body region; and
a coating of a fibrin binding agent on the body region.

In some embodiments, the fibrin binding agent selectively binds fibrin over fibrinogen.

In some embodiments, the fibrin binding agent comprises an antibody that specifically binds fibrin.

In some embodiments, the fibrin binding agent comprises a 59D8 antibody.

In some embodiments, the fibrin binding agent comprises a peptide that specifically binds fibrin, preferably a CREKA peptide.

In some embodiments, the clot-retrieval device comprises a stentriever.

In some embodiments, the clot-retrieval device comprises a self-expanding NITINOL body.

In some embodiments, the coating comprises a polydopamine (PDA) coating.

The invention also relates to a method of forming an implantable medical device, the method comprising:
functionalizing a nickel titanium surface of a body of an implantable medical device with a protein by: exposing the surface to a sodium hydroxide (NaOH) solution to hydroxylate the surface, rinsing the surface, and incubating the surface with a protein in a 2-Morpholinoethanesulfonic acid (MES) buffer to covalently link the protein to the nickel titanium surface.

In some embodiments, exposing the surface to the NaOH solution to hydroxylate the surface comprises exposing the surface to a solution of between 1M and 9M NaOH at between 70 and 90 degrees C for between 20 minutes and 3 hours,

In some embodiments, exposing the surface to the NaOH solution comprises exposing the surface to a 5M NaOH solution at 80 degrees C for 1 hour.

In some embodiments, the protein is a fibrin binding agent.

In some embodiments, the protein is an antibody.

In some embodiments, the protein comprises a 59D8 antibody.

In some embodiments, the protein comprises a CREKA peptide.

In some embodiments, incubating the surface with a protein comprises incubating with NHS (N-hydroxysuccinimide) and EDC (1-ethyl-3-(3-dimethylaminopropyl) carbodiimide) and the protein.

In some embodiments, the implantable medical device comprises a stent.

The invention relates also to a method of removing a clot from a body, the method comprising:
inserting a clot retrieval device into a blood vessel lumen, wherein the clot retrieval device comprises a fibrin binding protein bound via a covalent ester bond directly to a nickel titanium surface of the clot retrieval device;
attaching, by the fibrin binding protein, the clot to the clot retrieval device; and
removing the clot from the body.

In some embodiments, the method further comprises, after inserting the clot retrieval device, expanding the clot retrieval device in the blood vessel lumen.

In some embodiments, the method further comprises, prior to inserting the clot retrieval device, identifying that the patient has a clot blockage.

In some embodiments, the fibrin binding agent comprises a fibrin binding peptide, and attaching comprises attaching the clot to the fibrin binding peptide.

In some embodiments, the fibrin binding agent comprises an anti-fibrin antibody, and attaching comprises attaching the clot to the anti-fibrin antibody.

The invention also relates to a method of removing a clot from blood, comprising:
inserting a clot retrieval device comprising a coating of a fibrin binding agent into a blood vessel lumen; and
attaching, by the fibrin binding agent, the clot to the clot retrieval device.

In some embodiments, the method further comprises, after the inserting a clot retrieval device step, expanding the clot retrieval device in the blood vessel lumen.

In some embodiments, the method further comprises, prior to the inserting step, identifying that the patient is suspected of having a clot blockage.

In some embodiments, the fibrin binding agent comprises a fibrin binding peptide, and the step of attaching comprises attaching the clot to the fibrin binding peptide.

In some embodiments, the fibrin binding agent comprises an anti-fibrin antibody, and the step of attaching comprises attaching the clot to the anti-fibrin antibody.

The invention also relates to a method of forming a medical device, the method comprising:
deprotonating native and/or induced hydroxyl's on a nickel titanium surface of the medical device to form reactive hydroxyl group;
activate a carboxyl group (-COOH) on a protein to form an activated amide complex and stabilizing the activated amide complex;
forming a covalent ester bond between the reactive hydroxyl group and the activated amide complex to form a covalent ester bond linking the protein to the nickel titanium surface.

In some embodiments, deprotonating native and/or induced hydroxyl's on the nickel titanium surface comprises exposing the nickel titanium surface to a NaOH solution.

In some embodiments, activating the carboxyl group (-COOH) comprises using 1-ethyl-3-(3-dimethylaminopropyl) carbodiimide (EDC) to activate the carboxyl group.

In some embodiments, the method further comprises using N-hydroxy succinimide (NHS) to stabilize the activated amide complex.

In some embodiments, the protein comprises an antibody that specifically binds fibrin.

In some embodiments, the protein comprises a 59D8 antibody.

In some embodiments, the protein comprises a CREKA peptide.

In some embodiments, the medical device comprises a coil.

In some embodiments, the medical device comprises a stent.

In some embodiments, the medical device comprises a clot retriever.

The invention further relates to a method of forming an implantable medical device, the method comprising:
coating a body of an implantable medical device with a coating of a fibrin binding agent, wherein the coating is between 0.1 µm and 500 µm thick.

In some embodiments, coating targets fibrin but does not appreciably bind to fibrinogen.

In some embodiments, the method further comprised incubating the coated body at an alkaline pH.

In some embodiments, incubating the coated body at an alkaline pH comprises incubating in a bicarbonate buffer having a pH of about 9.

In some embodiments, coating the body comprises coating with a monoclonal antibody against fibrin, preferably a 59D8 antibody.

In some embodiments, coating the body comprises coating with a peptide that specifically binds fibrin, preferably a CREKA peptide.

In some embodiments, coating comprises dip coating the body.

In some embodiments, coating comprises coating in a polydopamine (PDA) coating.

In some embodiments, coating the body of the implantable medical device comprises coating onto a nickel-titanium surface.

In some embodiments, coating comprises coating in a polydopamine (PDA) coating and then coating with an anti-fibrin antibody or antibody binding fragment.

In some embodiments, coating comprises coating with NHS (N-hydroxysuccinimide) and EDC (1-ethyl-3-(3-dimethylaminopropyl) carbodiimide) and anti-fibrin antibody, anti-fibrin antibody binding fragment or a fibrin binding agent.

In some embodiments, coating comprises covalently attaching the fibrin binding agent to the implantable medical device.

The invention further relates to a method of treating an aneurysm, comprising:
inserting an insertion device into a blood vessel in need of treatment, wherein the insertion device carries a clot retrieval device comprising a coating of a fibrin binding agent; and
removing the insertion device to thereby leave the clot retrieval device in the blood vessel.

In some embodiments, the method further comprises attaching, by the fibrin binding agent, clot to the clot retrieval device.

In some embodiments, the coating comprises a fibrin binding protein covalently bound via a covalent ester bond directly to a nickel titanium surface of the clot retrieval device.

The invention relates to an endovascular clot-retrieval device, the device comprising:
a body region comprising a surface formed of a nickel titanium alloy; and
a fibrin binding protein covalently bound via a covalent ester bond directly to the surface formed of the nickel titanium alloy.

In some embodiments, (i) the fibrin binding protein selectively binds fibrin over fibrinogen, (ii) the fibrin binding protein comprises an antibody that specifically binds fibrin, (iii) the fibrin binding protein comprises a 59D8 antibody, (iv) the fibrin binding protein comprises a peptide that specifically binds fibrin, and/or (v) the fibrin binding protein comprises a CREKA peptide.

In some embodiments, the clot-retrieval device comprises a stentriever.

In some embodiments, the clot-retrieval device comprises a self-expanding nickel-titanium body.

The invention further relates to an endovascular clot-retrieval device comprising:
a body region; and
a coating of a fibrin binding agent on the body region.

In some embodiments, (i) the fibrin binding agent selectively binds fibrin over fibrinogen, (ii) the fibrin binding agent comprises an antibody that specifically binds fibrin, (iii) the fibrin binding agent comprises a 59D8 antibody, and/or (iv) the fibrin binding agent comprises a peptide that specifically binds fibrin, preferably a CREKA peptide.

The invention also relates to A method of forming an implantable medical device, the method comprising:
functionalizing a nickel titanium surface of a body of an implantable medical device with a protein by: exposing the surface to a sodium hydroxide (NaOH) solution to hydroxylate the surface, rinsing the surface, and incubating the surface with a protein in a 2-Morpholinoethanesulfonic acid (MES) buffer to covalently link the protein to the nickel titanium surface.

In some embodiments, exposing the surface to the NaOH solution to hydroxylate the surface comprises exposing the surface to a solution of between 1M and 9M NaOH at between 70 and 90 degrees C for between 20 minutes and 3 hours, preferably a 5M NaOH solution at 80 degrees C for 1 hour.

In some embodiments, the protein is a fibrin binding agent, preferably wherein the protein is an antibody, such as a 59D8 antibody, or preferably the protein comprises a CREKA peptide.

In some embodiments, incubating the surface with a protein comprises incubating with NHS (N-hydroxysuccinimide) and EDC (1-ethyl-3-(3-dimethylaminopropyl) carbodiimide) and the protein.

In some embodiments, the implantable medical device comprises a stent.

A method of forming a medical device, the method comprising:
deprotonating native and/or induced hydroxyl's on a nickel titanium surface of the medical device to form reactive hydroxyl group, preferably wherein deprotonating native and/or induced hydroxyl's on the nickel titanium surface comprises exposing the nickel titanium surface to a NaOH solution;
activating a carboxyl group (-COOH) on a protein to form an activated amide complex and stabilizing the activated amide complex, preferably wherein activating the carboxyl group (-COOH) comprises using 1-ethyl-3-(3-dimethylaminopropyl) carbodiimide (EDC) to activate the carboxyl group;
forming a covalent ester bond between the reactive hydroxyl group and the activated amide complex to form a covalent ester bond linking the protein to the nickel titanium surface.

In some embodiments, the method further comprises using N-hydroxy succinimide (NHS) to stabilize the activated amide complex.

In some embodiments, protein comprises (i) an antibody that specifically binds fibrin, preferably a 59D8 antibody; or (ii) a peptide that specifically binds fibrin, preferably a CREKA peptide.

In some embodiments, the medical device comprises a coil, a stent and/or a clot retriever.

### BRIEF DESCRIPTION OF THE DRAWINGS

A better understanding of the features and advantages of the methods and apparatuses described herein will be obtained by reference to the following detailed description that sets forth illustrative embodiments, and the accompanying drawings of which:
FIG. 1 illustrates the basic scheme of fibrin clot formation and fibrinolysis and the balance between these processes.
FIG. 2 shows fibrin filter assembly into a fiber network.
FIG. 3 illustrates the structure of an antibody, including the hypervariable and antigen binding sites, which may be used in the devices and methods herein.
FIGS. 4A-4F illustrate results on a test disc of nickel titanium for: control conditions (FIGS. 4A-4B), hydroxylation via peroxide under various conditions (FIGS. 4C-4E) and hydroxylation via an alkaline treatment (FIG. 4F) as described herein.
FIG. 5A is a graph comparing the various surface treatments and covalent binding of a test protein (Streptavidin) as described herein.
FIG. 5B is a graph quantifying the results of covalently binding an antibody (a mouse IgG) to a disk of nickel titanium using hydroxylation of the nickel titanium via an alkaline treatment (NaOH) and direct grafting of the antibodies as described herein.
FIGS. 6A-6D show images of test discs of nickel titanium following an alkaline (NaOH) treatment to show the effect of the use of a coupling agent such as EDC/NHS.
FIG. 7 is a graph illustrating the effect of alkaline (NaOH) treatments with and without a coupling agent, similar to the data shown in FIGS. 6A-6D.
FIG. 8 shows an example of a stent (e.g., part of a stentriever) that may be functionalized as described herein.
FIGS. 9A-9F illustrate 500x magnification views of untreated control stents (FIGS. 9A-9C) and functionalized/treated stents (FIGS. 9D-9F).
FIG. 10A shows an example of a control stent (showing no florescence, and thus no covalent binding of the surface to an antibody). FIG. 10B shows an example of a stent showing fluorescence indication that the nickel titanium surface has been directly and covalently bonded to an antibody (fluorescently labeled with a secondary, e.g., anti-mouse, antibody).
FIG. 11 is a graph showing antibody grafting directly to the surface of a stent using by hydroxylation of the nickel titanium surface of the stent via an alkaline treatment (NaOH).
FIGS. 12A-12B show a native stent (FIG. 12A) and a stent functionalized with an antibody that does not bind to clot (FIG. 12B) in an assay in which the stent is exposed to fluorescently labeled fibrin. FIGS. 12A-12B show no significant binding to fibrin. In contrast, FIG. 12C shows a stent that has been functionalized by hydroxylation of the nickel titanium surface of the stent via an alkaline treatment (NaOH) to include direct covalently linked anti-fibrin antibody (e.g., 59D8 anti-fibrin antibody), showing significant binding to clot material (e.g., fibrin).
FIG. 12D graphically illustrates the results of the assays shown in FIGS. 12A-12C, illustrating significant fibrin binding by stents functionalized to include anti-fibrin antibodies as descried herein.

### DETAILED DESCRIPTION

It would be particularly beneficial to provide surface modification (e.g., coatings) that may be readily used with nickel titanium medical devices, such as (but not limited to) clot retrieval devices, coils, and stents. It would be particularly beneficial to coat such medical devices with a polypeptide (e.g., protein), including antibodies and binding proteins. Such coatings may be used, for example, to provide anti-thrombogenicity and facilitate rapid endothelialization by coating with proteins that inhibit or prevent clot formation. Alternatively, coatings may provide local pro-thrombogenicity and facility binding to clot material by coating with proteins that enhance clot formation. Previous attempts at coating proteins to medical devices have met with limited success and/or require complex procedures, often resulting in relatively low coating densities or require one or more intermediate compounds to hold the protein to the nickel titanium surface.

The apparatuses (e.g., devices) described herein provide medical devices to which proteins are covalently attached directly to the nickel titanium surface of the medical device. For example, described herein are stents including a covalently attached coating of anti-fibrin proteins (e.g., fibrin-binding polypeptides including anti-fibrin antibodies). Also described herein are methods of coating a nickel titanium medical device (e.g., a clot retriever, coil, stent, etc.) by covalently attaching a protein directly to the nickel titanium.

For example, the devices and methods herein specifically target a major and specific component of thrombi, fibrin. Fibrin containing clots can attach to an endovascular device coated with a fibrin binding agent as described herein and then the clot and endovascular clot-retrieval device removed from a patient. This can result in reduced fragmentation of clots and their travel to other parts of the patient. Thus, described herein are devices and methods that incorporate fibrin-binding polypeptides into a coating on the device. These devices can modulate the engagement of a clot with the device, e.g., an insertable/implantable medical device such as a clot retriever, coil, stent, etc., in some cases creating a chemical/adhesive link in addition to the mechanical engagement provided by the design of the device. The fibrin-binding coating may provide an adhesive property to an endovascular device (e.g., stent bars/wires), which, in addition to their mechanical trapping effect, can result in an improved extraction of a thrombus.

Including molecules specific to fibrin vs. fibrinogen in an endovascular device can assist in better removing clot with less disruption of other blood functions, for example encouraging the formation of clot (and subsequent binding to clot material), which may be helpful, for example, with clot removal devices and/or occlusive coils (e.g., embolization coils). In some cases medical devices (e.g., stents) may including protein coatings that may inhibit clot formation and therefore reduce the likelihood of restenosis.

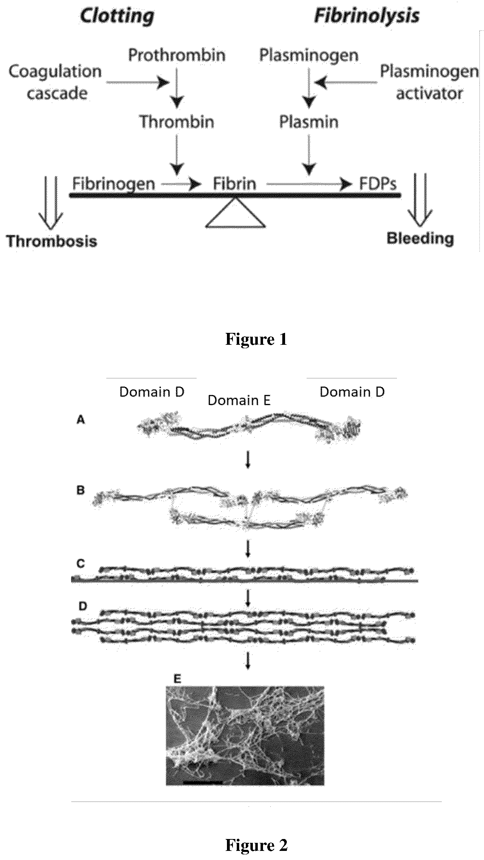

Fibrin is the main fibrillar component of all thrombi and is created in the last step of the coagulation cascade. The last step of the coagulation cascade is the transition of fibrinogen to fibrin (see, e.g., FIG. 1). Fibrin has a 3D organization different from that of fibrinogen and displays specific antigens. Thrombosis is a common cause leading to ischemic stroke and the conversion of fibrinogen to fibrin in the coagulation cascade leads to thrombosis, the formation of a clot that blocks the flow of blood through a vessel (thrombus). The coagulation system is of course critically important to stopping excessive bleeding or rebleeding caused by trauma, but in blood vessels coagulation can be highly dangerous. Thus, there is a delicate balance of components present in the blood stream and mechanical removal of clot tends to not upset the balance. Fibrin (factor Ia) is a long, thin protein with branches produced at the end of the coagulation cascade when fibrinogen (factor I) is converted to fibrin, which stabilizes the blood clot. Fibrinogen is a soluble blood protein that converts to an insoluble protein hydrogel clot, fibrin. A challenge in treating clots in the vessel is that the fibrin therein is an extremely extensible polymer and can be severely stretched without breaking.

One challenge is how to best remove blood clots without upsetting the delicate balance of components present in the blood stream. FIG. 2 illustrates fibrin filter assembly into a fiber network. In FIG. 2 the fibrin fiber assembly (A) is shown, including various fibrin domains. FIG. 2 also illustrates fibrinogen monomer (B), 46 nm long and 4.5 nm in diameter, composed of six polypeptide chains, and illustrates (C) fibrin molecules assemble longitudinally into half-stitches to form a protofibril made of two strands. In FIG. 2 the interactions are shown in yellow (small arrows). Protofibrils (E) aggregate laterally to form fibers, and branching leads to a network of fibers.

Naturally occurring peptides, proteins, and enzymes with fibrin-binding activity that bind to fibrin are known, some of which do not bind to fibrinogen. Such peptides have been used in imaging systems for rheumatoid arthritis, autoimmune disorders, or sepsis. Fibrin can also be a biomarker for central nervous system disorders or as an oncological marker for targeted cancer treatments. Fibrin has not previously been used for clot removal. Examples of such peptides that specifically target fibrin but not fibrinogen include monoclonal antibody 59D8 and the CREKA peptide. (see p.442 of Biophysical Reviews (2022) - "mouse immunization and hybridoma cell line development yielded the 59D8 mAb, which was found to bind human fibrin in the presence of fibrinogen. The monoclonal antibody 59D8 is one example of several known molecules that are specific for binding to fibrin but not fibrinogen, and which may be used in any of the devices and methods herein. 59D8 is a monoclonal antibody that specifically detects human and murine fibrin.

### Definitions:

Antibody. The term antibody (or immunoglobulin) encompasses polyclonal and monoclonal antibody preparations where the antibody may be of any class of interest (e.g., IgG, IgM, IgA, IgD, and IgE, and subclasses thereof), as well as preparations including hybrid antibodies, altered antibodies, F(ab')2 fragments, F(ab) molecules, Fv fragments, scFv fragments, single chain antibodies, single domain antibodies, chimeric antibodies, humanized antibodies, and functional fragments thereof which exhibit immunological binding properties of the parent antibody molecule, unless context indicates otherwise.

Some fragments can be produced by digestion with various peptidases. For example, pepsin digests an antibody below the disulfide linkages in the hinge region to produce F(ab)'2, a dimer of Fab which itself is a light chain joined to VH-CHI by a disulfide bond. While various antibody fragments are defined in terms of the digestion of an intact antibody, one of skill will appreciate that such fragments (e.g., Fab fragments) may be synthesized de novo either chemically or by utilizing recombinant DNA methodology. Thus, the term antibody, as used herein, also includes antibody fragments either produced by the modification of whole antibodies or synthesized de novo using recombinant DNA methodologies, including, but are not limited to, Fab'2, IgG, IgM, IgA, scFv, dAb, nanobodies, unibodies, and diabodies. These antibodies may be polyvalent forms (e.g., having duplicated binding domains.

A native antibody typically encompasses a protein having one or more polypeptides that can be genetically encodable. e.g., by immunoglobulin genes or fragments of immunoglobulin genes. Recognized immunoglobulin genes include the kappa, lambda, alpha, gamma, delta, epsilon and mu constant region genes, as well as myriad immunoglobulin variable region genes.

A typical immunoglobulin (antibody) structural unit is a tetramer. Each tetramer is composed of two identical pairs of polypeptide chains, each pair having one "light" (about 25 kD) and one "heavy" chain (about 50-70 kD). Light chains are classified as kappa or lambda. Heavy chains are classified as gamma, mu, alpha, delta, or epsilon, which in turn define the immunoglobulin classes, IgG, IgM, IgA, IgD, and IgE, respectively. The N-terminus of each chain defines a variable region of about 100 to 110 or more amino acids primarily responsible for antigen recognition. The terms variable light chain (VL) and variable heavy chain (VH) refer to these light and heavy chains respectively. A native antibody typically has a tetrameric structure. A tetramer typically comprises two identical pairs of polypeptide chains, each pair having one light chain (in certain embodiments, about 25 kDa) and one heavy chain (in certain embodiments, about 50-70 kDa). In a native antibody, a heavy chain comprises a variable region, VH, and three constant regions, CH1, CH2, and CH3. The VH domain is at the amino-terminus of the heavy chain, and the CH3 domain is at the carboxy-terminus. In a native antibody, a light chain comprises a variable region, VL, and a constant region, CL. The variable region of the light chain is at the amino-terminus of the light chain. In a native antibody, the variable regions of each light/heavy chain pair typically form the antigen binding site. The constant regions are typically responsible for effector function. In some variations, antibodies and fragments thereof encompass those that are bispecific. Bispecific antibodies may have binding specificities for at least two different epitopes, at least one of which can be an epitope of fibrin. For example, an antibody can be a fibrin-binding antibody, such as 59D8.

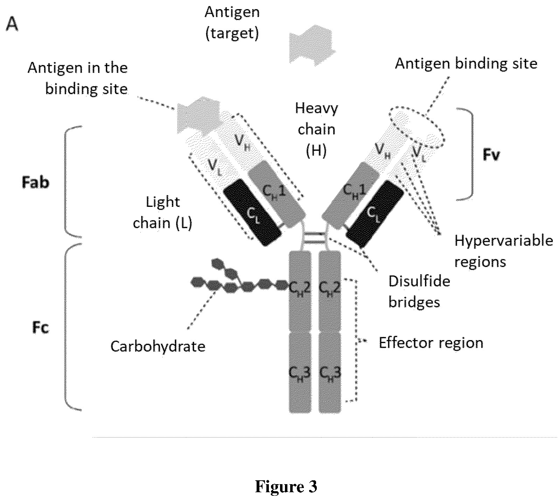

FIG. 3 illustrates the structure of an antibody, including the hypervariable and antigen binding sites. In FIG. 3 the antibody (such as an immunoglobulin G (IgG)) includes two heavy chains and two light chains connected by disulfide bridges. The specificity of the antibody for its antigen is determined by its variable fragment (Fv), while its effector functions are carried out by the constant region (Fc). The portion containing the antigen-binding site is called the "antigen-binding fragment" (Fab), which is formed by a light chain and a half-heavy chain. The devices, systems, and methods herein may utilize an antibody in its entirety or may utilize only a portion of an antibody, such as part or all of an antigen binding site, Fv fragment, Fab, etc.

An antigen-binding site or binding portion refers to the part of an antibody (for an antigen-binding site or other molecule that participates in antigen or other specific molecule binding. An antigen binding site in a native antibody is typically formed by amino acid residues of the N-terminal variable ("V") regions of the heavy ("H") and light ("L") chains.

A protein refers to a polypeptide of any appropriate length, including pentamers such as the CREKA protein.

"CREKA" refers to a protein (or region of a protein) having the sequence of amino acid Seq ID NO: 1 CREKA (Cys-Arg-Glu-Lys-Ala). A simple form of CREKA is a CREKA peptide. CREKA peptide (Cys-Arg-Glu-Lys-Ala) is a synthetic pentapeptide with a specific affinity for fibrin and fibrin-rich extracellular structures, especially those found in tumor microenvironments and thrombotic lesions. This specificity relies on the peptide's ability to recognize fibrin fibers formed within blood clots and tumor tissues, where it can accumulate in a targeted manner. The CREKA peptide has a structure stabilized by a cysteine (Cys) residue at the N-terminal position. This residue can form disulfide bonds, thereby enhancing the stability and adhesion of the peptide to fibrin. Its specific arrangement promotes the recognition of fibrin deposits while minimizing interactions with other plasma proteins. A CREKA peptide targets fibrin-rich matrices, making it a valuable tool for diagnostic and therapeutic applications. The interaction of the CREKA peptide with fibrin involves electrostatic forces and specific interactions with sites exposed during fibrin polymerization. This affinity has been demonstrated in several studies using thrombosis and fibrin-rich tumor models, where CREKA selectively binds to fibrin deposits and locates blood clots. See Zhang et al., J. of Nanobiology, 2023 Mar 3; 21:77; PMCID: PMC9985238; Zhang et al., Biomaterials, Volume 79, February 2016, Pages 46-55. The CREKA peptide (Cys-Arg-Glu-Lys-Ala) was discovered by in vivo screening of phage-displayed peptide libraries for tumor homing in transgenic cancer mice and shows specificity for fibrin over fibrinogen. See e.g., Zhang et al. Journal of Nanobiotechnology (2023) 21:77; doi.org/10.1186/s12951-023-01827-0; Risser et al. Biophysical Reviews (2022) 14:427-461; doi.org/10.1007/s12551-022-00950-w. Some variations of the CREKA peptide include longer peptides which include one or one or more CREKA peptide region(s). Longer peptides with a CREKA region can be 6 or more amino acids in length, 7 or more amino acids in length, 8 or more amino acids in length, 9 or more amino acids in length, 10 or more amino acids in length, more than 20 amino acids in length, more than 30 amino acids in length, etc. Some variations of the CREKA peptide can include conservative amino acid substitutions in the CREKA region. As used herein, CREKA peptide variations recognize fibrin fibers. CREKA peptide (CAS No. 847058-45-1) is available from MedChemExpress, Cat. No.: HY-P10709, Monmouth Junction, NJ, USA). A CREKA peptide can be represented as the following structure or a variation thereof:

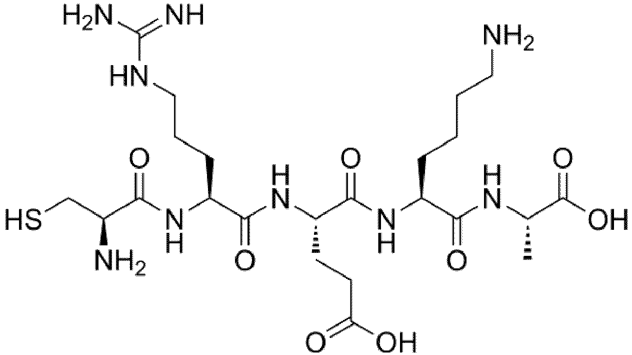

A fibrin specific peptide refers to a peptide (e.g., short protein composed of 10
amino acids) that specifically binds fibrin. In some particular examples, a fibrin specific peptide specifically binds fibrin over fibrinogen (by a 2-fold, a 10-fold, a 100-fold, etc.) difference. An example of a fibrin specific peptide is the CREKA peptide and variations thereof.

A coating refers to a layer of a substance on a substrate. A coating can be uniform or discontinuous. A coating may be, for example, up to 500 microns (or more) thick (e.g., up to 10, 15, 20, 40, 50 75, 100, 150, 250, and 500-microns including ranges herein (e.g., between 1-100 microns, between 1-250 microns, etc.).

In some examples a medical device such as a stent and/or clot-retrieval device may be evenly coated with a fibrin-binding agent or unevenly coated. Particular areas of a medical device may be coated with a fibrin agent (e.g., anti-fibrin antibody or CREKA protein) and other areas may not be coated. For example, a device such as a stentriever may have an inner surface and an outer surface may be coated with a fibrin binding agent on an inner surface and may not (or may) be coated with a fibrin binding agent on an outer surface. For example, a clot retrieval device may be roughly in shape of a hollow cylinder, and an inner surface of the clot retrieval device is the surface that faces an inside of the hollow cylinder. This may be the case even if the clot retrieval device has regions of discontinuity (e.g., composed of a series of wires).

An "epitope" is a site on an antigen (e.g. fibrin) to which an antibody binds. Epitopes can be formed both from contiguous amino acids or noncontiguous amino acids juxtaposed by tertiary folding of a protein.

Fibrin (Factor Ia) is a long, thin protein with branches produced at the end of the coagulation cascade when fibrinogen (factor I) is converted to fibrin, which stabilizes the blood clot. Fibrin, as a key protein in blood coagulation, plays a central role in stabilizing clots and promoting the progression of various pathologies, including thrombosis and certain cancers. Fibrin is an insoluble fibrous protein formed by the conversion of fibrinogen under the action of thrombin. This process leads to the polymerization of fibrin monomers into a three-dimensional network stabilized by factor XIIIa (FXIIIa or fibrin stabilizing factor). This network forms the main structure of blood clots and plays a crucial role in haemostasis by stopping bleeding and initiating the tissue healing process. The formation of fibrin begins with the cleavage of fibrinopeptides A and B from fibrinogen, exposing specific sites that favor the self-assembly of fibrin monomers. Once polymerized, fibrin is stabilized by covalent bonds formed by the action of transglutaminase factor XIIIa, which gives the clot its strength and mechanical resistance.

Fibrinogen is a soluble blood protein that converts to an insoluble protein hydrogel clot, fibrin.

As used herein, a fibrin binding agent refers to an agent that specifically binds to fibrin. Examples of fibrin binding agents include anti-fibrin antibodies and certain small peptides (such as CREKA). See e.g., Kolodziej et al. "Fibrin Specific Peptides Derived by Phage Display: Characterization of Peptides and Conjugates for Imaging", Bioconjug Chem. 2012 March 21; 23(3): 548-556. doi:10.1021/bc200613e. A fibrin binding agent may be referred to an anti-fibrin agent.

Fibrinogen is a 340kDa hexameric plasma glycoprotein. Fibrinogen is synthesized by the liver, circulates in the blood, and is a major structural component of a clot.

High affinity. The term "high affinity" when used with respect to an antibody refers to an antibody that specifically binds to its target(s) with an affinity (KD) of at least about 10⁻⁷ M at least about 10⁻⁸ M, preferably at least about 10⁻⁹ M, at least about 10⁻¹⁰ M, and at least about 10⁻¹¹ M. "High affinity" antibodies may have a KD ( equilibrium dissociation constant, a ratio of koff/kon, between the antibody and its antigen) that ranges from about 1 nM to about 0.01 pM.

Specificity refers to the ability of a molecule (e.g., an antibody) to recognize and bind its intended epitope and to distinguish between different potential ligands. A fibrin specific molecule (antibody) may be specific for fibrin molecules, such as found in a blood clot. Non-limiting examples of fibrin specific antibodies useful for the purposes herein include mouse monoclonal antibodies 59D8, 55D10, and 64C5 (Hui et al. Science, 9 Dec 1983, Vol 222, Issue 4628, pp. 1129-1132 DOI: 10.1126/science.6648524) and monoclonal antibody T2G1(Kudryk BJ, Grossman ZD, McAfee JG, Rosebrough SF. Monoclonal antibodies as probes for fibrin(ogen) proteolysis. In: Chatal J-F, ed. Monoclonal Antibodies in Immunoscintigraphy. Boca Raton, FL: CRC Press; 1989:365-398) and variations thereof. Antibody specificity can be measured by, for example, a screening test against different related antigens using ELISA, RIA, Western blotting, dot blotting, and such as described in Hui et al. In some examples, a fibrin binding agent selectively binds fibrin over fibrinogen.

The term "RH899201 antibody" refers to an anti-fibrin antibody, such as to the monoclonal anti-fibrin antibody, clone 59D8. Clone 59D8 is an IgG1 antibody specifically located at the N-terminal end of the beta chain of human fibrin. This epitope is exposed only after the cleavage of fibrinopeptide B from fibrinogen by thrombin, which gives this antibody high specificity for fibrin while not recognizing circulating fibrinogen. The monoclonal antibody 59D8 was produced using a synthetic heptapeptide corresponding to the N-terminal end of the beta chain of human fibrin as the antigen. It has been shown that this antibody can specifically bind to fibrin even in the presence of physiological concentrations of fibrinogen in plasma. Moreover, its anti-fibrin activity was inhibited by its peptide antigen but not by a control heptapeptide, confirming the specificity of its binding. Several studies have shown that the 59D8 antibody recognizes both human and canine fibrin but not bovine, ovine, or porcine fibrin. This selectivity is explained by the presence of a leucine residue at position 5 of the beta chain of human fibrin, which plays a key role in recognition by the antibody. This leucine residue at position 5 is a structural determinant allowing antibody 59D8 to differentiate fibrins from different species. The antibody was generated by immunization with the human beta (1-7) peptide having the sequence of amino acid Seq ID NO: 2 GHRPLDK (Gly-His-Arg-Pro-Leu-Asp-Lys), and it was observed that this sequence is specific to human and canine fibrins but differs in other species. The RH899201 antibody is available from ProteoGenix (Newark, DE, USA; Schiltigheim, France).

### Devices

Described herein are medical devices, including implants, formed at least in part of a nickel titanium alloy (e.g., NITINOL). These device may include a covalently (and directly) attached protein, including but not limited to, fibrin-binding proteins. These devices may include stents and clot retrieval devices. For example a clot retrieval device can be an endovascular clot-retrieval device including a body region; and a coating of a fibrin binding agent on the body region. The coating of fibrin binding protein can be a fibrin binding agent that selectively binds fibrin over fibrinogen. The coating of fibrin binding agent can be a covalently attached antibody that specifically binds fibrin. The fibrin binding agent can include a peptide that specifically binds fibrin. In some embodiments, the coating of the fibrin binding agent can be coated at a concentration (e.g., coating density) of greater than 0.1 mg/m² (e.g., greater than 0.5 mg/m², greater than 1 mg/m², greater than 1 mg/m², etc.).

The clot retrieval device herein can be a coated stentreiver. Typical stentriever geometry may be coil, spiral, woven, individual rings, or sequential, essentially any structure that can be configured into a tubular, (and often) self-expandable configuration. Stentreivers can be single layer or multiple layers. Stentrievers can be formed as a sheet, tubing, wire, and ribbon or flat wire forms. Sheet-based stents are typically rolled into a tubular configuration after the pattern has been formed. Stentrievers may be used alone or in tandem with direct aspiration devices. Stentriever may be made of a shape-memory material, such as NITINOL. The clot-retrieval device may include a self-expanding NITINOL body. Examples of commercially available stentriever devices usable with the techniques and methods herein include, but are not limited to, Catchview^{™} revascularization device (Balt Extrusion SAS), Solitaire^{™} flow restoration device (Medtronic), Trevo^{™} retriever (Stryker), and Eric^{™} retrieval device (Terumo Neurovascular). Catchview is a NITINOL laser-cut closed cell design, self-expandable and resheathable, with a consistent radial force, overlapping design, longitudinal slit, and an antikinking pusher.

Some embodiments of a clot-retrieval device may be self-expanding. A clot-retrieval device may be configured to engage and integrate the clot material, even given varying clot composition and density. For example, because the proposed coating selectively adheres to fibrin, which is present in clots of varying composition and density, it typically leads to increased engagement of clot with device. Some embodiments of a clot-retrieval device are configured to be re-sheathed and removed while minimizing clot breakage and distal embolization of clot material. Some embodiments of a clot-retrieval can have high rates of first-pass recanalization (ability to remove clot and restore flow on the first deployment) which may be attributed to engagement by a chemical in addition to mechanical engagement, thus increasing the high rate of first pass removal. Some embodiments of a clot-retrieval device can result in reduced amounts of endoluminal damage to the vessel wall during deployment and retrieval (as compared e.g., to a comparable clot-retrieval device without a fibrin binding agent). Some embodiments of a clot-retrieval device are configured for multiple re-deployments in the target vessel or more distally in the case of incomplete or failed clot material.

In some variations, a clot-retrieval device can be a coil, microvention web, or another mesh or mesh-like structure. A clot-retrieval device can facilitate clot generation thereon and may be useful for aneurysm treatment. A clot-retrieval device with clots thereon may block blood flow. A clot-retrieval device may prevent an aneurysm from rupturing.

Other devices may include implants such as stents, coils (e.g., embolization coils), etc.

### Methods

Methods of making and using these medical devices, and in particular methods of coating these medical devices by forming a direct covalent bond to the surface of the nickel titanium, are described herein. In general, described herein are methods of attaching an agent such as an anti-fibrin agent to a medical device. For example, described herein are methods of making a medical device such as a clot retrieval device by covalently and directly attaching a protein (such as a fibrin binding agent) to the device. In some cases the clot-retrieval agent (e.g., anti-fibrin agent) may be grafted onto a clot retrieval device. A clot-retrieval agent may be attached covalently or non-covalently onto a clot retrieval device.

Methods of coating a device with a protein agent such as a fibrin-binding protein may include, but are not limited to, chemical vapor deposition (CVD), dip coating (dipping), electrochemical deposition (electrodeposition), and spray coating. With CVD, a precursor gas is introduced into a chamber and reacts to form a coating on the surface of the substrate. With dipping, the devices can be submerged in a container of coating and then withdrawn for drying and post-coating operations. With electrodeposition, an electric current is used to deposit a material onto a clot retrieval device, such as including but not limited to those described herein. With spray coating, the coating material is atomized and applied to the substrate in a fine spray. For example, dip coating may be performed using polydopamine (PDA) dip coating. A device can be treated with one or more suitable formulations or another treatment to provide desired properties on a device. A device can be treated with a formulation or another treatment prior to treatment of the device with a clot-retrieval agent, simultaneous with treatment with a clot-retrieval agent (the formulation can include a clot-retrieval agent), or after treatment with a clot retrieval agent. A device can be treated with one, two, or more formulations or other treatments. A formulation or other treatment applied to a device can add desirable properties, such as biocompatibility, lubriciousness, etc. A formulation or other treatment applied to a device can enable or facilitate binding a clot-retrieval agent to a device. Coating a device (such as a NITINOL device) with a desired polymer can enhance the biocompatibility of the device's surface properties, thus enabling the addition of a fibrin binding agent, such as a fibrin binding peptide or fibrin binding antibody. For example, methods of coating a device include coating a device with polydopamine. In some examples, methods of coating a device include coating a device with norepinephrine, poly(ethylamine)-catechol, chitosan-catechol, another catechol containing polymer, polypyrrole, etc.

Other device treatments can enable or facilitate binding of a clot-retrieval agent such as a fibrin binding agent to a device. For example, a device can be treated with hydrogen peroxide (H₂O₂). A device can be treated with an alkaline treatment and then a clot-retrieval agent such as a fibrin binding agent such as an antibody or peptide as described herein. A device can be treated with one or more of an alkaline treatment an NHS (N-hydroxy succinimide) treatment, and an EDC (1-ethyl-3-(3-dimethylaminopropyl) carbodiimide) treatment. A device can be treated with one or more of a polydopamine (PDA) treatment and click chemistry treatment. A device can be treated with one or more of a polydopamine (PDA) treatment and click chemistry treatment, such as strain-promoted alkyne-azide cycloaddition (SPAAC).

Thus, methods of preparing a device may include a step of treating the device with hydrogen peroxide (H₂O₂) or and alkaline treatment and an NHS (N-hydroxy succinimide) and EDC (1-ethyl-3-(3-dimethylaminopropyl) carbodiimide) treatments. Any of these treatments can include or be followed by treatment with a clot-retrieval agent such as described herein (antibody, CREKA peptide, etc.) or otherwise known antibody. In some variations, more than one treatment (treatment formulations) can be utilized. For example, a second crosslinker can be used with a first PDA as a first layer on the clot retrieval device. In some variations, a clot retrieval device can be coated with a fibrin binding agent by incubating the device with the fibrin binding agent (e.g., antibody, peptide) at alkaline pH (0.1 M bicarbonate buffer pH 9).

Exemplary surface modification strategies can include one or more of the following. In some cases these techniques may direly covalently bond the protein to the nickel titanium surface by appropriately preparing the nickel titanium surface and reacting with EDC/NHS. The method in which the surface is prepared may be particularly important and may dramatically affect the amount and distribution of the protein covalently bound to the nickel titanium surface.

### Method 1: Hydroxylation of NITINOL with hydrogen peroxide and direct grafting of antibodies

NITINOL may be treated with hydrogen peroxide (H₂O₂) to increase corrosion resistance. This treatment promotes the formation of hydroxyl groups (Ti-OH) on the surface of NITINOL, however this treatment also makes the surface more reactive for subsequent chemical reactions, such as covalent grafting. H₂O₂ acts as a powerful oxidant; by exposing NITINOL to aqueous hydrogen peroxide solutions, oxidation occurs, forming a thicker titanium oxide (TiO₂) layer. This oxide layer can improve the chemical homogeneity of the surface and contribute to the protection of the material against corrosion. Hydrogen peroxide treatment can also slow down the dissolution of nickel, which can be an important factor in improving biocompatibility, as nickel leaching is one of the main issues associated with NITINOL in biomedical applications. The surface treatment of NITINOL with hydrogen peroxide has also been studied for its effects on biocompatibility. The surface modifications induced by hydrogen peroxide can reduce nickel leaching and thus improve the biocompatibility of NITINOL medical devices.

Antibodies contain carboxyl groups (-COOH) in their structure, particularly on the side chains of amino acids such as glutamic acid or aspartic acid. These groups can be used for chemical conjugation. NHS (N-hydroxysuccinimide) and EDC (1-ethyl-3-(3-dimethylaminopropyl) carbodiimide) can be used to activate carboxyl groups (-COOH). EDC activates the carboxyl group to form a reactive intermediate (an activated amide complex), and NHS stabilizes this intermediate, facilitating the reaction with a nucleophile, such as a hydroxyl (OH) group on a surface. Once the surface of the NITINOL is hydroxylated (with Ti-OH groups) and an antibody has been activated via NHS/EDC to expose a reactive group on its COOH, the two reactants can form a covalent ester bond. This reaction occurs through the interaction between the hydroxyl group on the NITINOL and the activated intermediate of the carboxyl group of the antibody, thus forming a stable ester. This may be summarized as:

| | |
|---|---|
| Hydroxylation: | NITINOL (TiNi) + H₂O₂ → TiOH + Ni oxide |
| Activation COOH: | Antibody-COOH + EDC + NHS → Antibody-COO-EDC |
| Functionalization: | Ti-OH + Anticorps-COO-EDC → Ti-O-C-Antibody + EDC |

Treatment of nickel titanium (e.g., NITINOL) with H₂O₂. solutions at 3% and 30% were performed, leading to the formation of a titanium oxide TiO₂ layer on the surface. This layer is enriched with hydroxyl groups (OH) which increases the chemical reactivity of the surface and serve as anchoring sites for the covalent grafting of biomolecules, thereby facilitating surface functionalization for biomedical applications. A slightly acidic buffer (MES, pH 6) was used. At a pH less than the pKa (approximately 4.5) the carboxyl is formed in its protonated from (-COOH, neutral); at pH greater than the pKa (approximately5), the group is deprotonated to -COO⁻ (negatively charged).

Preliminary result comparing 3% H₂O₂ to 30 H₂O₂ shows that although 3% H₂O₂ does not impact the nickel titanium surface, 30% H₂O₂ increases the roughness of the surface. Further, grafting of fluorescent streptavidin to the surface as described above did not result in substantial covalent bonding for either 30% H₂O₂ (80 degrees C for 1 or 2 hours) or 3% H₂O₂ (80 degrees C for 1 or 2 hours), though 3% was marginally better (n=3).

Hydroxylation may also be combined with HCl treatment and/or NaOH treatment. For example, H₂O₂ (30%) treatment as described above was also applied followed by treatment with 0.1M HCl. In an acidic environment, hydrogen peroxide reacts with titanium to form an amorphous oxide layer. With short treatment durations, a thin and porous TiO₂ film may be formed. The oxide layer thickness in a 30% H₂O₂ / 0.1 M HCl mixture increases linearly over time. Peroxide (H₂O₂) also generates reactive oxygen species that enhance the attack on metallic (e.g., nickel titanium) surfaces. Low-concentration HCl may help eliminate non-oxidized metal ions (e g Ni²⁺) and may clean the surface. This may lead to the formation of a hydrophilic titanium oxide layer with surface - OH groups (via partial hydrolysis of TiO₂). The resulting surface may be clean, reactive, and suitable for molecular grafting.

When applying this treatment to nitinol pellets, the procedure was performed it in two steps: first with H₂O₂, followed by a 0 1 M HCl solution. This sequential approach was chosen to modify the nitinol surface in a controlled manner, to facilitate streptavidin grafting. X-ray crystallography (e.g., XRD patterns) of specimens treated with an H₂O₂/HCl solution at 80°C for 30 min and subsequently heat-treated at various temperatures for 1 h, showed a transformation of crystal structure of the gels.

Similarly, sequential hydroxylation (H₂O₂) and mild NaOH treatment may also be performed. For example, 30% or 3% H₂O₂ treatment followed by NoOH (0.1 M) treatment may enrich surface -OH groups. After oxidation with H₂O₂ and formation of TiO, mild NaOH treatment may form a thin layer of sodium titanate (Na₂TiO₃), may expose more surface hydroxy (-OH) groups and/or may promote the ionization of Ti-OH into Ti-O⁻ , increasing reactivity for covalent grafting.

### Method 2: Hydroxylation of NITINOL via an alkaline treatment (NaOH) and direct grafting of antibodies

NaOH solution can provide a strongly alkaline environment that can deprotonate native (or induced) hydroxyls present on the surface of titanium oxide, forming reactive hydroxyl groups (-OH) on the surface. The latter becomes hydrophilic and functional for modifications such as chemical grafting (covalent bonds, etc.). The Ti - O - Ti bonds are broken under the action of NaOH, and hydroxide ions (OH-) attach to the exposed titanium atoms, generating Ti - OH groups. Antibodies, on the other hand, contain carboxyl groups (-COOH) in their structure, particularly on the side chains of amino acids such as glutamic acid or aspartic acid. These groups can be used for chemical conjugation.

NHS (N-hydroxy succinimide) and EDC (1-ethyl-3-(3-dimethylaminopropyl) carbodiimide) are commonly used to activate carboxyl groups (-COOH). EDC activates the carboxyl group to form a reactive intermediate (an activated amide complex), and NHS stabilizes this intermediate, facilitating the reaction with a nucleophile, such as an OH group on a surface. EDC/NHS may be optionally used with the NaOH treatments described herein. For example, once the surface of the NITINOL is hydroxylated (with Ti-OH groups) the antibody may be activated via NHS/EDC to expose a reactive group on its COOH, and the two reactants can form a covalent ester bond. This reaction occurs through the interaction between the hydroxyl group on the NITINOL and the activated intermediate of the carboxyl group of the antibody, thus forming a stable ester. The chemical steps of this reaction may be summarized by:

| | |
|---|---|
| Hydroxylation: | TiO₂ + NaOH → TiOH + NaO₂ |
| Activation COOH: | Antibody-COOH + EDC + NHS → Antibody-COO-EDC |
| Functionalization: | Ti-OH + Antibody-COO-EDC → Ti-O-C-Antibody + EDC |

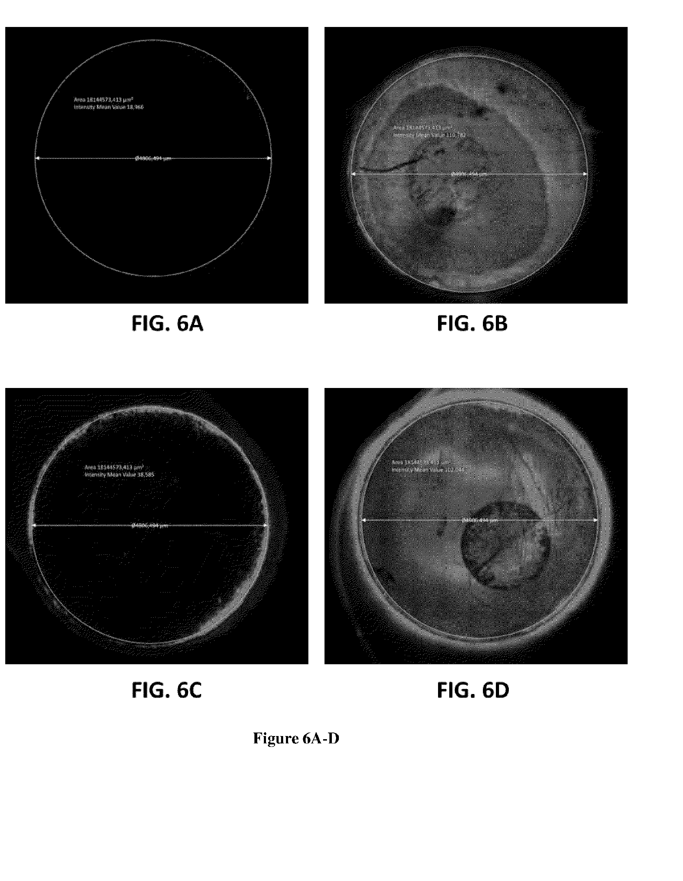

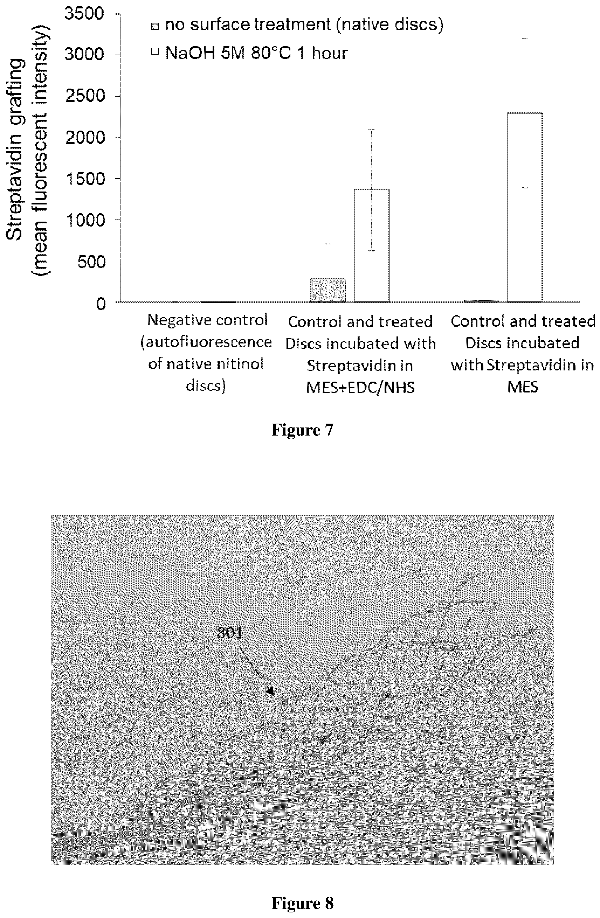

Surprisingly, NaOH treatment to covalently bond a protein may be performed without the use of EDC/NHS. For example, FIGS. 6A-6D show a comparison between discs of nickel titanium hydroxylation via an alkaline treatment (NaOH) with NHS/EDC and without NHS/EDC. FIGS. 6A-6B show the results of a 1 hour at 80°C treatment of a disc of nickel titanium with either 1M NaOH (FIG. 6A) or 5M NaOH (FIG. 6B) followed by NHS/EDC and Streptavidin 555. These treatments were repeated without the NHS/EDC, as shown in FIGS. 6C (1M NaOH) and FIG. 6D (5M NaOH). As also shown in the graph of FIG. 7, substantial grafting of protein to the nickel titanium was achieved following just the 5M NaOH alkaline treatment; thus alkaline treatment with 5 M NaOH enables the subsequent grafting of fluorescent streptavidin in MES buffer following irrespective of the use of a coupling agent such as EDC/NHS.

In addition, the use (or non-use) of a coupling agent such as EDC/NHS during the grafting step does not appear to disrupt the covalent binding between the nickel titanium surface and the protein (e.g., Streptavidin) as preliminary results show that substantial streptavidin is retained onto the nickel titanium surface upon prolonged exposure to whole blood.

This Alkaline NaOH treatment (e.g., with between 1 M and 10 M NaOH, such as 1M NaOH, 5M NaOH, etc.), and particularly treatment with concentrated sodium hydroxide, may create a nanostructured surface rich in Ti-OH groups on titanium and its alloys. This may result in the formation of titanium hydroxides (Ti-OH) on the surface that can serve as anchoring sites for chemical grafting. When TiO2 particles, e.g., in the rutile form, are treated with a concentrated NaOH solution, some Ti-O-Ti bonds are hydrolyzed, resulting in the formation of Ti-O-Na and Ti-O-H bonds. A high concentration of NaOH (e.g., 10M) and extended treatment times may be used to evaluate surface deformation and the formation of chemical groups. In some cases it may be beneficial to minimize surface deformation (porosity) and preserve the mechanical properties of the nitinol by minimizing the concentration of NaOH while optimizing for the functionalization of the resulting surface; thus, relatively lower concentrations (< 10M NaOH) and shorter treatment times (3 hours or less, 2 hours or less, 1.5 hours or less, 1 hour or less, etc.) may be used.

Micrographs of nickel titanium surfaces treated by hydroxylation via an alkaline treatment (NaOH) as described herein show that within the ranges described herein (e.g., between 1M and <10M) the surface is not significantly modified. For example, unlike peroxide treatments, treatment with NaOH at either 1 M or 5 M does not appear to modify the structure of the nitinol disc surface.

### Method 3: Treatment with polydopamine and conjugation with NHS/EDC

Polydopamine (PDA) is a polymer obtained by the auto-oxidation of dopamine, which contains high molar ratios of amino acid residues with primary amines (RNH₂) and catechol (C₆H₆O₂), motifs that contribute to robust interfacial adhesion. Polydopamine (PDA) is composed of dense hyperbranched oligomers that merge into colloidal particles in the overall solution and conformally coat interfaces, including substrates immersed in a buffer.

The carboxyl groups present on the antibodies can be activated via NHS/EDC and are converted into activated esters (NHS-ester) ready to react with the amine groups of polydopamine, forming a covalent amide bond. These chemical steps may be summarized as:

| | |
|---|---|
| Treatment polydopamine: | TiO₂+ Dopamine → TiO₂-PDA |
| Activation COOH: | Antibody-COOH + EDC + NHS → Antibody-COO-EDC |
| Functionalization: | Antibody PDA-NH₂ + Antibody-COO-EDC → PDA-NH-CO- |

### Method 4: Graft 3-Phosphonopropionic acid onto NITINOL to form a ligand, then bind antibodies via NHS-EDC conjugation.

The grafting of 3-Phosphonopropionic acid (3PPA) onto NITINOL can utilize a chemical interaction between the phosphonic groups (-PO(OH)_2) of 3PPA and the titanium oxide (TiO_2) layer naturally formed on the surface of NITINOL. This interaction allows for a strong chemical adsorption of 3PPA, involving the formation of Ti-O-P bonds, which stabilize the 3PPA molecule on the substrate.

The 3PPA, once fixed on the surface of the NITINOL, exposes its free carboxyl group (-COOH), which constitutes a reactive site for conjugation with antibodies. This conjugation is facilitated by a chemical activation using the pair N-hydroxy succinimide (NHS) and 1-ethyl-3-(3-dimethylaminopropyl) carbodiimide (EDC). EDC is a coupling agent that reacts with the carboxyl group of 3PPA to form an activated intermediate (O-acylisourea), which is then stabilized by NHS by forming an NHS ester. This activated ester facilitates the reaction with the amine groups (-NH_2) present on the antibodies, thereby allowing the formation of a stable covalent amide bond between the 3PPA and the antibodies. This may be summarized as:
Grafting of 3PPA on NITINOL:

   TiO₂ + HO-P(O)(OH)-CH2-CH2-COO → TiO₂-O-P(O)(OH)-CH₂-CH₂-COOH
Activation of the carboxyl groups of 3PPA via EDC/NHS:

   R-COOH + EDC → R-COO-EDC

   R-COO-EDC + NHS → R-COO-NHS + EDC
Conjugation with antibodies:

   R-COO-NHS + NH₂-Anticorps → R-CONH-Anticorps + NHS

Also described herein are methods that do not include the use of EDC/NHS, but may instead use click chemistry.

### Method 5: Treatment with polydopamine (PDA) followed by using click chemistry with a bi-orthogonal linker (DBCO) to graft the antibody

Copper-free click chemistry is based on the reaction of a DBCO fragment (Dibenzylcyclooctyne amine) with, for example, a reaction partner labeled with an azide, known as strain-promoted alkyne-azide cycloaddition (SPAAC). Unlike conventional click chemistry, this copper-free click chemistry is very fast at room temperature and does not require the Cu(I) catalyst (cytotoxic to most organisms), thus preventing its use in many biological systems. The treatment with polydopamine (PDA) introduces functional groups on the surface of NITINOL, which serve as anchoring points for a bi-orthogonal triple bond linker (e.g., DBCO or DBCO derivative). The linker then selectively reacts with a reagent containing an azide group in a click chemistry reaction, allowing the covalent grafting of antibodies (or other biomolecules) onto the surface of the substrate via a triazole bond. "Click chemistry" encompasses various chemical reactions resulting in the formation of covalent bonds under mild conditions and with nearly quantitative yields. These chemical steps may be summarized by:
Treatment polydopamine: TiO2 + Dopamine + alkaline solution → TiO2 + PDA
Click chemistry with DBCO: PDA-NH2 + DBCO-époxyde → PDA-DBCO
Azide marker on the antibody:Antibody-NH2 + Linker-N3 → Antibody-N3
Functionalization: PDA-DBCO + Antibody-N3 → PDA-DBCO-Antibody

Any of these coating methods may be used alone or in conjunction with other coating methods. The device (e.g., a clot retrieval device) may be coated in a sterile environment (e.g., manufactured under sterile conditions), and/or may be sterilized after manufacturing.

In some cases the second method, e.g., the method of covalently coating a protein to a nickel titanium surface using hydroxylation of nickel titanium via an alkaline treatment (NaOH) and EDC/NHS may be particularly useful. For example, table 1, below illustrates results of an assay in which a protein and a fluorophore (e.g., streptavidin conjugated to a fluorophore) was covalently linked to a disk of nickel titanium as described using one of the techniques described above.

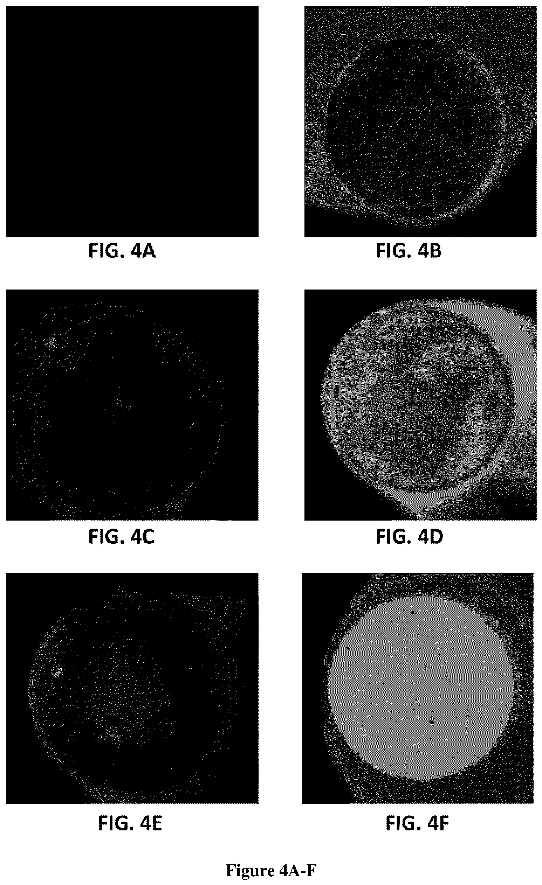

FIGS. 4A-4F illustrate a comparison of fluorescent streptavidin grafting to nickel titanium discs according to the hydroxylation protocol described above (e.g., method 2, above). FIG. 4A shows the negative control (showing very little auto fluorescence of native NITINOL). FIG. 4B is a first control showing a native disc of nickel titanium exposed to the Streptavidin 555 (e.g., streptavidin Alexa Fluor^{™} 555) in a 2-Morpholinoethanesulfonic acid (MES) buffer. FIG. 4C shows the results of treatment with 30% peroxide treatment for 1 hour at 80°C then NHS/EDC and Streptavidin 555 in MES buffer (method 1, above). Relatively low (if any) covalent bonding was seen. FIG. 4D shows the result of a treatment with 30% peroxide followed by exposure to Streptavidin 555 in MES buffer, resulting in some (non-uniform) surface functionalization/binding. FIG. 4E shows the results following treatment with 30% peroxide for 1 hour at 80°C followed immediately by 0.1M HCl and then NHS/EDC and Streptavidin 555 in MES buffer. Relatively little binding of the Streptavidin protein was seen. In contrast, FIG. 4F shows a highly significant amount of covalent binding of Streptavidin (Streptavidin 555) following treatment in a 5M NaOH solution at 80°C followed by NHS/EDC and Streptavidin 555 in MES buffer. FIG. 4F illustrates an examples of method 2, showing a protein (Streptavidin) covalently bound to a disk of nickel titanium using hydroxylation of the NITINOL via an alkaline treatment (NaOH) and direct grafting of antibodies with NHS/EDC.

For example in coating a nickel titanium surface of a medical device, the surface may be modified as described herein by immersion of the medical device (e.g., stent) in 5M NaOH at about 80°C for approximately 1 hour. For example, the temperature may be between 60°C and 90°C, e.g., between about 70°C and 90°C, etc. The medical device may then be washed (e.g., three times) for about 5 minutes by immersion in water (H₂O), and then incubation of the modified medical device with the protein or peptide of interest for 20 minutes or longer (e.g., between 5 minutes and 4 hours, between 10 minutes and 3 hours, between 10 minutes and 2 hours, between 10 minutes and 1 hour, between 10 minutes and 30 minutes, 5 minutes or longer, 10 minutes or longer, 15 minutes or longer, 20 minutes or longer, 30 minutes or longer, etc.) in 50 mM MES buffer at pH 6 (MES = 2-Morpholinoethanesulfonic acid). The medical device may then be washed, e.g., in water with a detergent, multiple times (e.g., 3 or more times) for about 5 minutes (e.g., one minute or longer, 2 minutes or longer, 3 minutes or longer, 4 minutes or longer, 5 minutes or longer, etc.). For example, the medical device may be washed in water supplemented with about 0.1% tween 20. Finally, the medical device may be rinsed in buffer one or more times (e.g., 2 or more times) for about 1 or more minutes each time (such as between 1-10 minutes, between 2-7 minutes, about 5 minutes, etc.) in PBS (phosphate buffer saline).

FIG. 4A shows the florescence after binding streptavidin (which has been conjugated with a fluorophore) with 30% hydrogen peroxide in an acidic condition (0.1M HCL) after 30 minutes at 80 degrees C. The average fluorescence was measured at an intensity of 54.771, as shown in the second row of table 1, below. FIG. 4B shows the florescence after binding streptavidin (conjugated with a fluorophore) with 5M NaOH alkaline medium and streptavidin activated via NHS/EDC conjugation, we obtain a homogeneous and uniform fluorescence. The average fluorescence intensity is 1471.206, as shown in the third row of table 1:

| | Sample | Average fluorescence intensity | Average intensity offset |
|---|---|---|---|
| 1 | 30% H₂O₂, 1 hr. + NHS/EDC + Streptavidin (AF 555) | 39.765 | 18.247 |
| 2 | 30% H₂O₂ + 0.1 M HCl, 30 min + NHS/EDC + Streptavidin (AF 555) | 54.771 | 33.253 |
| **3** | **5M NaOH, 1 hr. + NHS/EDC + Streptavidin (AF 555)** | **1471.206** | **1449.688** |
| 4 | 30% H₂O₂, 1 hr. + PDA + NHS/EDC + Streptavidin (AF 555) | 83.941 | 62.423 |
| 5 | 30% H₂O₂ + 0.1 M HCl, 30 min + PDA + NHS/EDC + Streptavidin (AF 555) | 81.570 | 60.052 |
| 6 | 5M NaOH, 1 hr. + PDA + NHS/EDC + Streptavidin (AF 555) | 160.073 | 138.555 |
| 7 | Control (NHS/EDC + Streptavidin (AF 555)) | 86.156 | 64.638 |
| 8 | Control (30% H₂O₂, 1 hr. + Streptavidin (AF 555)) | 275.132 | 253.614 |
| 9 | Control | 21.518 | 0 |

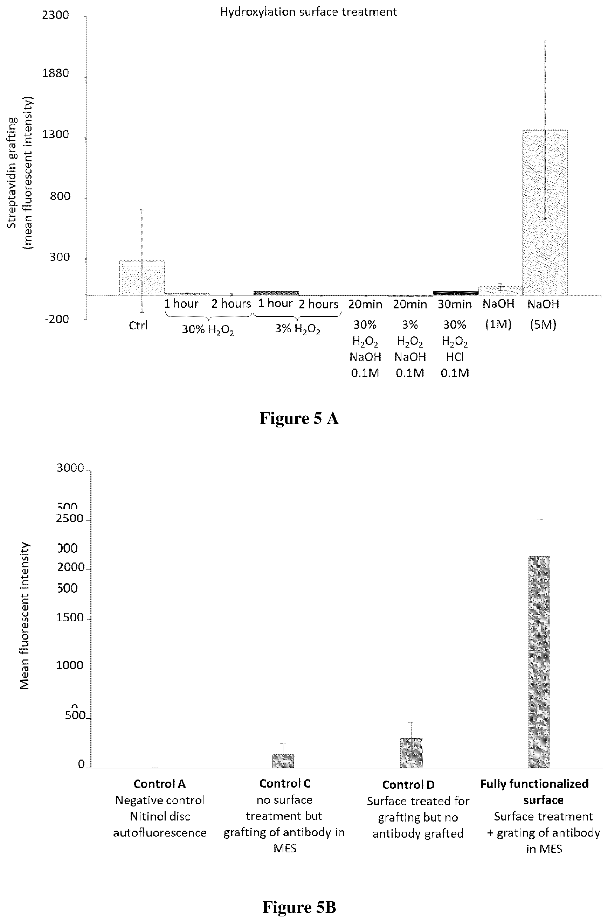

These results are also illustrated in the graphs shown in FIGS. 5A-5B. FIG. 5A shows a comparison of the efficiency of Streptavidin (AF 555) binding for various treatments described herein, as compared with control. As discussed above, the surface treatment with 5M NaOH for one hour was significantly better at grafting (covalently bonding) the protein (Streptavidin) to the nickel titanium than other methods, including as compared with 1M NaOH. Similarly, FIG. 5B shows results when using a mouse IgG antibody (and an anti-Mouse fluorescent conjugate), showing substantial antibody grafting to the surface as compared to control (including exposure to just the florescent label with and without surface modification, without the mouse IgG).

Both the intensity and distribution of the fluorescence signal using the hydroxylation of NITINOL with hydrogen peroxide and direct, covalent conjunction of protein with NHS/EDC showed a significant signal. The duration of the Hydroxylation and/or activation steps may be varied (e.g., one hour is shown in Table 1, but the time may be adjusted, e.g., between 1 minutes and 2 hours or more, 2.5 hour or more, 3 hours or more, 3.5 hours or more, 4 hours or more, 5 hour or more, 6 hours or more, etc.).

### Methods of using

Also described herein are methods of using any of these apparatuses. For example, methods of removing a clot from blood using a NITINOL clot retriever device coated with an anti-fibrin protein may include inserting the clot retrieval device into a blood vessel lumen and binding clot to the clot retrieval device via the anti-fibrin protein bound to an outer surface of the device. The method herein may also include expanding the clot retrieval device in the blood vessel lumen. The method may include identifying that the patient is suspected of having a clot blockage, e.g., prior to the inserting step. In some cases the anti-fibrin protein is a fibrin binding agent that comprises a fibrin binding peptide (e.g., CREKA), and the step of binding to clot material may include binding the clot/clot material to the fibrin binding peptide.

In any of these methods the anti-fibrin protein (e.g., the fibrin binding agent) comprises an anti-fibrin antibody, and binding may include binding clot to the anti-fibrin antibody and therefore the device. In some embodiments, the fibrin binding agent does not significantly interact with fibrinogen (i.e., does not specifically bind fibrinogen), but does specifically bind to fibrin. In some embodiments, the fibrin binding agent does not interact with (i.e., does not specifically bind) other material in the patient's blood other than fibrin/blood clots. A fibrin-binding agent may bind to fibrin with greater affinity (2 fold stronger, 10 fold stronger, 100 fold stronger, etc.) than it binds to fibrinogen or another material in patient's blood.

Also described herein are methods of treating an aneurysm. A method for treating an aneurysm may include one or more of the steps of inserting an insertion device into a blood vessel in need of treatment, wherein the insertion device carries a clot retrieval device comprising a coating of a fibrin binding agent; removing the insertion device to thereby leave the clot retrieval device in the blood vessel; and attaching, by the fibrin binding agent, clot to the clot retrieval device. An insertion device may be, for example, a catheter inserted into a blood vessel. A clot retrieval device for treating an aneurysm may be a coil, a mesh, a mesh-like structure, a web, etc.

### Examples

In one, non-limiting, example biological functionalization of a stent retriever was performed to improve mechanical thrombectomy, e.g., in the treatment of ischemic stroke. Mechanical thrombectomy may be enhanced to improve clot remove and retention of clot fragments and to reduce treatment time by covalently bonding peptides or proteins that bind to fibrin, which is present in all types of clot. For example, the methods and compositions described herein may generate chemical groups that allow the direct conjugation of anti-fibrin antibodies or peptides to metal struts of a stent (e.g., part of a stentriever) to form organometallic stents with clot-adhesive struts. The functionalized surfaces of these struts are not merely coatings or embedded materials but are conjugated directly with the surface material. This is achieved by generating hydroxyl (-OH) groups on the surface of the nickel titanium, generating a metal oxide nanomaterial intermediary.

In some cases, this may be achieved by alkaline treatment with NaOH within a range of conditions (e.g., as described above in reference to method 2), including temperature, concentration of the NoOH, and incubation buffer to allow conjugation of proteins to the hydroxylated NITINOL surface through reactions between the generated OH groups and groups present in the protein and amino acids to form covalent bonds, i.e., ester or amide bonds. The incubation buffer chosen should enable reactions such as the acylation of the alcohol, esterification and/or metal oxide-catalyzed amidation of proteins (e.g., antibodies) with the surface of the medical device (e.g., stent).

The methods described herein, including in particular method 2, the hydroxylation of nickel titanium via an alkaline treatment (NaOH) may be used for direct grafting of proteins, including antibodies. This was tested (as shown in FIG. 5) using both indirect testing, e.g., by covalently bonding a primary antibody to the nickel titanium surface and testing for binding with a probe secondary antibody to which a fluorophore has been coupled (e.g., a fluorescent secondary antibody). Alternatively, the antibody covalently bonded to the nickel titanium surface may be directly conjugated to a fluorophore or other indicator. Thus, 5M NaOH-treated nickel titanium surfaces may also graft directly to antibodies prepared in MES buffer. This is shown in FIG. 5B, discussed above. As shown with streptavidin, antibodies prepared in MES were also efficiently grafted to NaOH-treated surfaces of an implant.

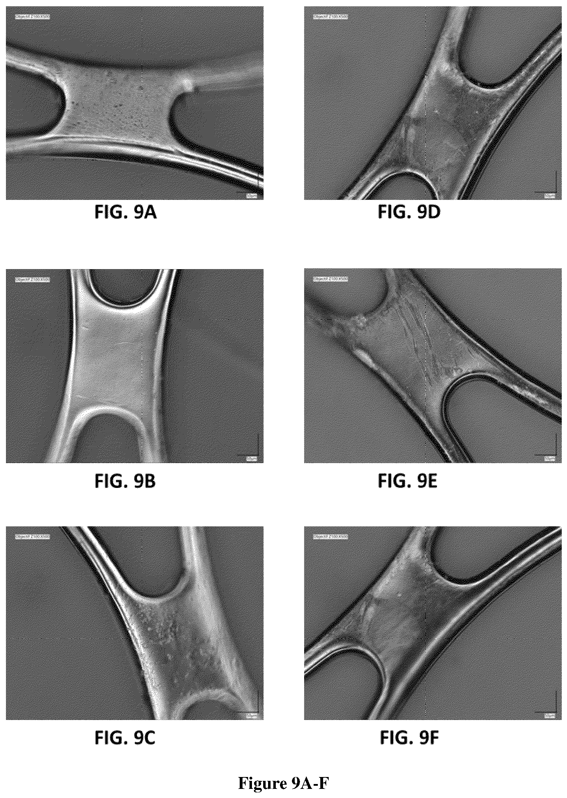

FIG. 8A shows an example of a nickel titanium stent (e.g., a laser-cut nickel-titanium stent) 801. FIGS. 9A-9F show enlarged images of a portion of this nickel titanium stent to show the effect of activation of the nickel titanium surface by alkaline treatment (NaOH). FIGS. 9A-9C show examples of surfaces that were not activated by alkaline treatment (NaOH), while FIGS. 9D-9F show surfaces that were active by alkaline treatment (NaOH), using 5 M NaOH for 1 hour at 80°C. No significant differences are apparent on the surface (e.g., at 500x magnification).

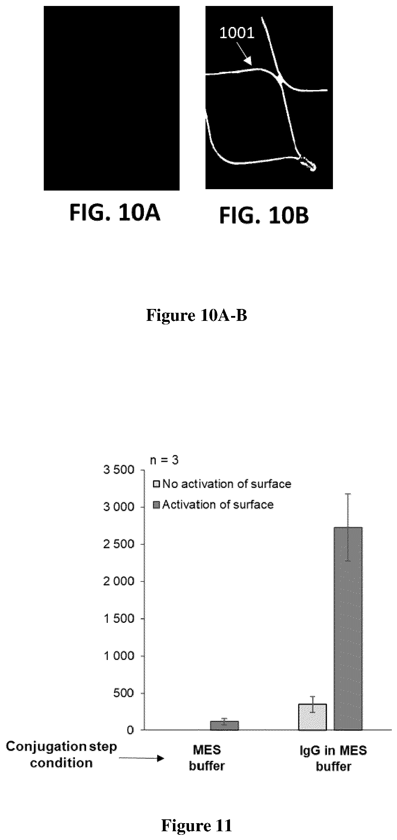

The alkaline treatment (NaOH), which may also be referred to as NaOH-MES buffer-based protein grafting, results in a substantial and long-lasting functionalization of the surface of the stent. This is illustrated in FIGS. 10A-10B and FIG. 11. Stents were treated with an alkaline treatment (NaOH) as described above (e.g., 1 hour at 80°C treatment with 5 M NaOH, wash with buffer, then incubating with IgG in MES buffer, followed by rinsing with PBS and exposure to fluorescently labeled secondary antibody to detect). FIG. 10A shows a typical control, showing no significant fluorescence either by autofluorescence, by MES buffer alone (no NaOH treatment), or by NaOH treatment without grafting to antibody. FIG. 10B shows a stent treated as with NaOH then exposure to antibody in MES buffer; the fluorescently labeled secondary antibody shows robust fluorescence, indicating covalent binding to the stent 1001. The graph shown in FIG. 11 shows the mean fluorescent intensity in control (no activation either with MES buffer or activation with just MES buffer but no antibody, similar to FIG. 10A) and activation with IgG in MES buffer (similar to FIG. 10B).

The covalent binding of antibody to the nickel titanium surfaces of stents also appears to be stable over time, and after exposure to mechanical manipulation (e.g., deployment from an introducer sheath, including at least 5-6 back-and-forth passes into/out of an introducer sheath). Thus, the grafting appears stable after mechanical manipulation.

Stents treated with an alkaline treatment (5M NaOH) as described herein to covalently bond a protein (e.g., antibody) to the nickel titanium surface have an effectively functionalized surface. For example, an anti-fibrin antibody (e.g., 59D8 anti-fibrin antibody) was grafted (e.g., covalently bound) directly to the nickel titanium surface of a stent using an alkaline treatment (NaOH) as described herein. In this example, stents functionalized by alkaline treatment (5M NaOH) to covalently bind to anti-fibrin antibodies were found to induce fibrin clot formation and binding to the stent *in vitro.* Citrated, platelet-poor plasma (PPP) was supplemented with fluorescent fibrinogen and coagulation was induced by addition of CaCl₂ (recalcification). Functionalized stents were incubated (37 °C for 1 hour) with PPP. Fluorescent fibrin clot was sonicated to partially disrupt the structure and then incubated with the stents for 30 minutes, and the stent was washed in PBS.

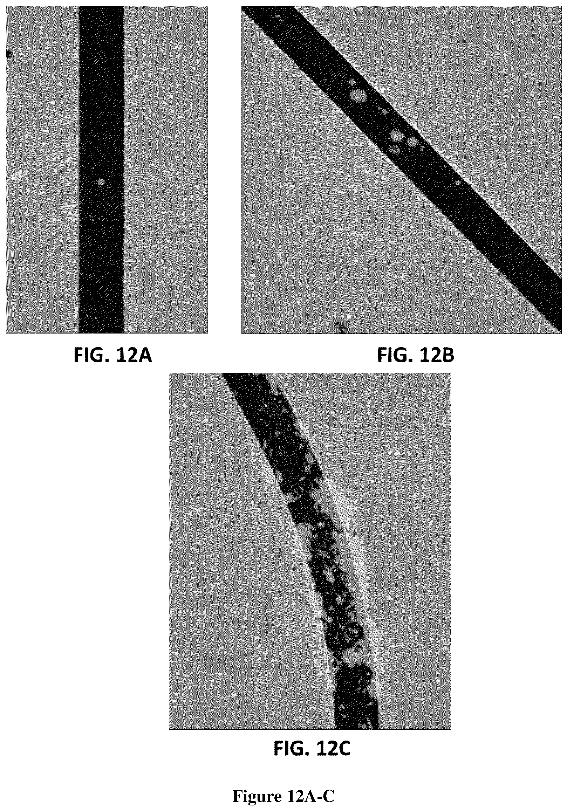

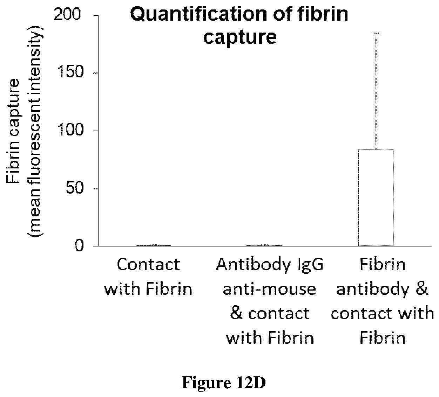

FIGS. 12A-12D illustrate images showing the biological efficacy of stents directly functionalized with anti-fibrin antibody in this assay. FIG. 12A shows the native stent (not functionalized) with virtually no fluorescence from a plasma clot. FIG. 12B illustrates capture of fluorescent fibrin by a stent functionalized with an irrelevant control antibody (very little florescence seen). FIG. 12C shows capture of fluorescent fibrin from a plasma clot by a stent functionalized with an anti- fibrin antibody. As shown graphically in FIG. 12D, while control native stents do not interact with fibrin, stents functionalized with the anti-fibrin antibody capture fibrin on the stent.

Thus in this example, chemical treatment of nickel titanium surfaces with NaOH (e.g., between 1M-10M, at between about 70-90 °C, for about 0.5-3 hours) enables effective surface activation for subsequent conjugation of proteins and antibodies prepared in MES buffer. In the particular example shown about, organometallic stents may be formed by alkaline (NaOH) treatment that does not require a coupling agent such as NHS/EDC and provides mechanically and biologically stable grafting of proteins onto the nickel titanium surfaces. For example, the 59D8 anti-fibrin antibody may be grafted by this method onto stents, will retain its functionality, and confers the stent the ability to capture fibrin from clots.

All publications and patent applications mentioned in this specification are herein incorporated by reference in their entirety to the same extent as if each individual publication or patent application was specifically and individually indicated to be incorporated by reference. Furthermore, it should be appreciated that all combinations of the foregoing concepts and additional concepts discussed in greater detail below (provided such concepts are not mutually inconsistent) are contemplated as being part of the inventive subject matter disclosed herein and may be used to achieve the benefits described herein.

When a feature or element is herein referred to as being "on" another feature or element, it can be directly on the other feature or element, or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under", or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

In general, any of the apparatuses and methods described herein should be understood to be inclusive, but all or a sub-set of the components and/or steps may alternatively be exclusive and may be expressed as "consisting of" or alternatively "consisting essentially of" the various components, steps, sub-components or sub-steps.

"An embodiment," "in an embodiment", "in another embodiment," "embodiments," "certain embodiments," or "in some embodiments" each has the meaning herein of "in one or more embodiments". Similarly, "a configuration," "in a configuration," in one configuration," "in another configuration," "configurations," "certain configurations," or "in some configurations" each has the meaning herein of "in one or more configurations."

The terms "e.g.," "such as," "for example," "and so forth," and "etc." mean that what follows the "e.g.," "such as," or "for example" or what precedes the "etc." or "and so forth," is a list of examples and there may be other items that could also serve as examples but are not listed.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/-10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

### SEQUENCE LISTING

SEQ ID NO: 1 CREKA peptide (Cys-Arg-Glu-Lys-Ala):
   CREKA
SEQ ID NO: 2 human beta (1-7) peptide (Gly-His-Arg-Pro-Leu-Asp-Lys):
   GHRPLDK

## Claims

1. An endovascular clot-retrieval device, the device comprising:
a body region comprising a surface formed of a nickel titanium alloy; and
a fibrin binding protein covalently bound via a covalent ester bond directly to the surface formed of the nickel titanium alloy.

2. The device of claim **1,** wherein (i) the fibrin binding protein selectively binds fibrin over fibrinogen, (ii) wherein the fibrin binding protein comprises an antibody that specifically binds fibrin, (iii) wherein the fibrin binding protein comprises a 59D8 antibody, (iv) wherein the fibrin binding protein comprises a peptide that specifically binds fibrin, and/or (v) wherein the fibrin binding protein comprises a CREKA peptide.

3. The device of claim **1** or claim **2,** wherein the clot-retrieval device comprises a stentriever.

4. The device according to any one of claims **1** to **3,** wherein the clot-retrieval device comprises a self-expanding nickel-titanium body.

5. An endovascular clot-retrieval device comprising:
a body region; and
a coating of a fibrin binding agent on the body region.

6. The endovascular clot-retrieval device of claim **5,** wherein (i) the fibrin binding agent selectively binds fibrin over fibrinogen, (ii) wherein the fibrin binding agent comprises an antibody that specifically binds fibrin, (iii) wherein the fibrin binding agent comprises a 59D8 antibody, and/or (iv) wherein the fibrin binding agent comprises a peptide that specifically binds fibrin, preferably a CREKA peptide.

7. A method of forming an implantable medical device, the method comprising:
functionalizing a nickel titanium surface of a body of an implantable medical device with a protein by: exposing the surface to a sodium hydroxide (NaOH) solution to hydroxylate the surface, rinsing the surface, and incubating the surface with a protein in a 2-Morpholinoethanesulfonic acid (MES) buffer to covalently link the protein to the nickel titanium surface.

8. The method of claim **7,** wherein exposing the surface to the NaOH solution to hydroxylate the surface comprises exposing the surface to a solution of between 1M and 9M NaOH at between 70 and 90 degrees C for between 20 minutes and 3 hours, preferably a 5M NaOH solution at 80 degrees C for 1 hour.

9. The method of claim **7** or claim **8,** wherein the protein is a fibrin binding agent, preferably wherein the protein is an antibody, such as a 59D8 antibody, or preferably the protein comprises a CREKA peptide.

10. The method of any one of claims **7** to **9,** wherein incubating the surface with a protein comprises incubating with NHS (N-hydroxysuccinimide) and EDC (1-ethyl-3-(3-dimethylaminopropyl) carbodiimide) and the protein.

11. The method of any one of claims **7** to **10,** wherein the implantable medical device comprises a stent.

12. A method of forming a medical device, the method comprising:
deprotonating native and/or induced hydroxyl's on a nickel titanium surface of the medical device to form reactive hydroxyl group, preferably wherein deprotonating native and/or induced hydroxyl's on the nickel titanium surface comprises exposing the nickel titanium surface to a NaOH solution;
activating a carboxyl group (-COOH) on a protein to form an activated amide complex and stabilizing the activated amide complex, preferably wherein activating the carboxyl group (-COOH) comprises using 1-ethyl-3-(3-dimethylaminopropyl) carbodiimide (EDC) to activate the carboxyl group;
forming a covalent ester bond between the reactive hydroxyl group and the activated amide complex to form a covalent ester bond linking the protein to the nickel titanium surface.

13. The method of claim **12,** further comprising using N-hydroxy succinimide (NHS) to stabilize the activated amide complex.

14. The method of claim **12** or claim **13,** wherein the protein comprises (i) an antibody that specifically binds fibrin, preferably a 59D8 antibody; or (ii) a peptide that specifically binds fibrin, preferably a CREKA peptide.

15. The method of any one of claims **12** to **14,** wherein the medical device comprises a coil, a stent and/or a clot retriever.
